(19) Europäisches Patentamt
European Patent Office
Office européen des brevets

(11) Veröffentlichungsnummer: **0 213 129 B1**

# (12) EUROPÄISCHE PATENTSCHRIFT

(45) Veröffentlichungstag der Patentschrift: **11.11.92**

(51) Int. Cl.5: **C07C 405/00**, A61K 31/557

(21) Anmeldenummer: **85901971.3**

(22) Anmeldetag: **12.04.85**

(86) Internationale Anmeldenummer:
**PCT/DE85/00120**

(87) Internationale Veröffentlichungsnummer:
**WO 85/04656 (24.10.85 85/23)**

Die Akte enthält technische Angaben, die nach dem Eingang der Anmeldung eingereicht wurden und die nicht in dieser Patentschrift enthalten sind.

(54) **NEUE 9-HALOGEN-PROSTAGLANDINE.**

(30) Priorität: **13.04.84 DE 3414509**

(43) Veröffentlichungstag der Anmeldung:
**11.03.87 Patentblatt 87/11**

(45) Bekanntmachung des Hinweises auf die Patenterteilung:
**11.11.92 Patentblatt 92/46**

(84) Benannte Vertragsstaaten:
**AT BE CH DE FR GB IT LI LU NL SE**

(56) Entgegenhaltungen:
**EP-A- 0 008 003**
**EP-A- 0 030 377**
**FR-A- 2 206 096**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **RADÜCHEL, Bernd**
**Gollanczstr. 132**
**W-1000 Berlin 28(DE)**
Erfinder: **SKUBALLA, Werner**
**Olwenstr. 13**
**W-1000 Berlin 28(DE)**
Erfinder: **VORBRÜGGEN, Helmut**
**Wilkestr. 7**
**W-1000 Berlin 27(DE)**
Erfinder: **LOGE, Olaf**
**Bekassinen Weg 37**
**W-1000 Berlin 27(DE)**
Erfinder: **ELGER, Walter**
**Schorlemer Allee 12b**
**W-1000 Berlin 27(DE)**

EP 0 213 129 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aus dem Europäischen Patent 30 371 sind 9-Chlorprostaglandinderivate mit gesättigter oberer Seitenkette sowie einer cis-Doppelbindung in 5-Position bekannt.

In FR 2206096 werden 9-Keto- sowie 9-Hydroxy-prostaglandinderivate mit 4,5-ungesättigter oberer Seitenkette beschrieben.

Aus der Europäischen Patentanmeldung 8003 sind 4,5- ungesättigte 16-Phenoxy-9-ketoprostaglandine bekannt.

Die vorliegende Erfindung betrifft neue 9-Halogenprostaglandinderivate, Verfahren zu ihrer Herstellung sowie diese enthaltende Arzneimittel.

Aus dem sehr umfangreichen Stand der Technik der Prostaglandine und ihrer Analoga weiß man, daß diese Stoffklasse aufgrund ihrer biologischen und pharmakologischen Eigenschaften zur Behandlung von Säugetieren, einschließlich des Menschen, geeignet ist. Ihre Verwendung als Arzneimittel stößt jedoch häufig auf Schwierigkeiten. Die meisten natürlichen Prostaglandine besitzen eine für therapeutische Zwecke zu kurze Wirkungsdauer, da sie zu rasch durch verschiedene enzymatische Prozesse metabolisch abgebaut werden. Alle Strukturveränderungen haben das Ziel, die Wirkungsdauer sowie die Selektivität der Wirksamkeit zu steigern.

Es wurde nun gefunden, daß die neuen 9-Halogen-prostaglandinderivate eine hervorragende Wirkungsspezifität, bessere Wirksamkeit, längere Wirkungsdauer als natürliche Prostaglandine und deren Derivate besitzen und besonders für die orale Applikation geeignet sind.

Die Erfindung betrifft 9-Halogen-prostanderivate der Formel I

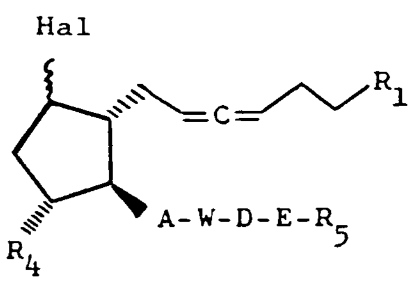

(I),

worin Hal ein α- oder ß-ständiges Chlor- oder Fluoratom,

$R_1$ den Rest $CH_2OH$ oder

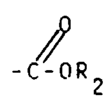

mit $R_2$ in der Bedeutung eines Wasserstoffatoms, eines $C_1$-$C_4$-Alkylrestes, welcher gegebenenfalls ein- bis mehrfach durch Fluor, Chlor, Brom, Phenyl, Benzoyl, Dimethylamino, Diethylamino, Methoxy oder Ethoxy substituiert ist, eines $C_5$-$C_6$-Cycloalkylrestes, oder $R_2$ Phenyl, 1-Naphthyl oder 2-Naphthyl bedeutet,
die jeweils substituiert sein können
durch 1-3 Halogenatome, eine Phenylgruppe, 1-3 Alkylgruppen mit jeweils 1-4 C-Atomen, eine Chlormethyl-, Fluormethyl-, Trifluormethyl-, Carboxyl-Hydroxy- oder Alkoxygruppe mit 1-4 C-Atomen, oder 2-Furyl, 2-Thienyl, 2-Pyridyl, 3-Pyridyl, 4-Pyridyl, Oxazolyl, Thiazolyl, Pyrimidinyl, Pyridazinyl, 3-Furyl, 3-Thienyl, 2-Tetrazolyl oder
$R_1$ den Rest

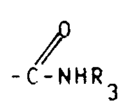

mit $R_3$ in der Bedeutung eines organischen Carbonsäure- oder Sulfonsäurerestes mit 1-15 C-Atomen oder des Restes $R_2$ und

A eine $-CH_2-CH_2-$, eine trans-CH = CH- oder eine -C≡C-Gruppe,

W eine freie oder durch Tetrahydropyranyl, Tetrahydrofuranyl, $\alpha$-Äthoxyathyl, Trimethylsilyl, Dimethyl-tert.-.butyl-silyl, Tribenzylsilyl, Acetyl, Propionyl, Butyryl und Benzoyl verätherte oder veresterte Hydroxymethylengruppe oder eine freie oder durch Tetrahydropyranyl, Tetrahydrofuranyl, $\alpha$-Äthoxyäthyl, Trimethylsilyl, Dimethyl-tert.-butyl-silyl, Acetyl, Propionyl, Butyryl und Benzoyl verätherte oder veresterte

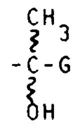 Gruppe,

wobei die OH-Gruppe $\alpha$- oder $\beta$-ständig sein kann,

D und E gemeinsam eine direkte Bindung oder

D eine geradkettige, verzweigte oder ringförmige Alkylengruppe mit 1-10 C-Atomen, die gegebenenfalls durch Fluoratome substituiert ist, und

E ein Sauerstoff- oder Schwefelatom, eine direkte Bindung, eine -C≡C-Bindung oder eine $-CR_6 = CR_7$-Gruppe darstellt, wobei $R_6$ und $R_7$ sich unterscheiden und ein Wasserstoffatom, ein Chloratom oder eine $C_1$-$C_4$-Alkylgruppe bedeuten,

$R_4$ eine freie oder durch Tetrahydropyranyl, Tetrahydrofuranyl $\alpha$-Äthoxyäthyl, Trimethylsilyl, Dimethyl-tert.-.butyl-silyl, Tribenzylsilyl, Acetyl, Propionyl, Butyryl und Benzoyl verätherte oder veresterte Hydroxygruppe,

$R_5$ ein Wasserstoffatom, eine Alkyl- oder Alkenylgruppe mit bis zu 6 C-Atomen, eine Halogensubstituierte Alkyl- oder Alkenylgruppe mit bis zu 6 C-Atomen, eine $C_3$-$C_6$-Cycloalkylgruppe, Phenyl, 1-Naphthyl und 2-Naphthyl, die jeweils substituiert sein können durch 1-3 Halogenatome, eine Phenylgruppe, 1-3 Alkylgruppen mit jeweils 1-4 C-Atomen, eine Chlormethyl-, Fluormethyl-, Trifluormethyl-, Carboxyl-, Hydroxy- oder Alkoxygruppe mit 1-4 C-Atomen oder $R_5$ 2-Furyl, 2-Thienyl, 2-Pyridyl, 3-Pyridyl, 4-Pyridyl, Oxazolyl, Thiazolyl, Pyrimidinyl, Pyridazinyl, Pyrazinyl, 3-Furyl, 3-Thienyl bedeuteu, und falls $R_2$ die Bedeutung eines Wasserstoffatoms hat, deren Salze mit physiologisch verträglichen Basen.

Als Alkylgruppen $R_2$ sind gerade oder verzweigte Alkylgruppen mit 1- 4 C-Atomen zu betrachten, wie beispielsweise Methyl, Äthyl, Propyl, Butyl, Isobutyl, tert.-Butyl, Die Alkylgruppen $R_2$ können gegebenenfalls ein- bis mehrfach substituiert sein, wobei die einfache Substitution bevorzugt sein soll. Als bevorzugte Alkylgruppen $R_2$ sind z.B. Methyl, Äthyl, Propyl, Dimethylaminopropyl, Isobutyl, Butyl zu nennen.

Als Arylgruppen $R_2$ kommen sowohl substituierte wie auch unsubstituierte Arylgruppen in Betracht. Bevorzugt sind die Substituenten in 3- und 4-Stellung am Phenylring, zum Beispiel Fluor, Chlor, Alkoxy oder Trifluormethyl oder in 4-Stellung Hydroxy.

Die Cycloalkylgruppe $R_2$ kann im Ring 5 oder 6 Kohlenstoffatome enthalten. Die Ringe können durch Alkylgruppen mit 1-4 Kohlenstoffatomen substituiert sein. Beispielsweise seien genannt Cyclopentyl-, Cyclohexyl und Methylcyclohexyl.

Als Säurerest $R_3$ sind physiologisch verträgliche organische Carbonsäuren und Sulfonsäuren mit 1-15 Kohlenstoffatomen geeignet, die der aliphatischen, cycloaliphatischen, aromatischen, aromatisch-aliphatischen und heterocyclischen Reihe angehören. Diese Säuren können gesättigte, ungesättigte und/oder mehrbasisch und/oder in üblicher Weise substituiert sein. Als Beispiele für die Substituenten seien Alkyl-, Hydroxy-, Alkoxy-, Oxo- oder Aminogruppen oder Halogenatome erwähnt. Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diäthylessigsäure, tert.-Butylessigsäure, Cyclopropylessigsäure, Cyclopentylessigsäure, Cyclohexylessigsäure, Cyclopropancarbonsäure, Cyclohexancarbonsäure, Phenylessigsäure, Phenoxyessigsäure, Methoxyessigsäure, Äthoxyessigsäure, Mono-, Di- und Trichloressigsäure, Aminoessigsäure, Diäthylaminoessigsäure, Piperidinoessigsäure, Morpholinoessigsäure, Milchsäure, Bernsteinsäure, Adipinsäure, Benzoesäure, mit Halogen-, Trifluormethyl-, Hydroxy-, Alkoxy- oder Carboxy-Gruppen substituierte Benzoesäuren, Nikotinsäure, Isonikotinsäure, Furan-2-carbonsäure, Cyclopentylpropionsäure. Als bevorzugte Acylreste werden solche mit bis zu 10 Kohlenstoffatomen betrachtet. Als Sulfonsäuren kommen beispielsweise Alkansulfonsäuren mit 1-10 C-Atomen wie z.B. Methansulfonsäure, Äthansulfonsäure, Isopropansulfonsäure und Butansulfonsäure sowie $\beta$-Chloräthansulfonsäure, Cyclopentansulfonsäure, Cyclohexansulfonsäure, Benzolsulfonsäure, p-Toluolsulfonsäure, p-Chlorbenzolsulfonsäure, N.N-Dimethylaminosulfonsäure, N.N-Diäthylaminosulfonsäure, N.N-Bis-(β-chloräthyl)-aminosinlfonsäure, N.N-Diisobutylaminosulfonsäure, N.N-Dibutylaminosulfonsäure, Pyrrolidino-, Piperidino-, Piperazino-, N-Methylpiperazino- und Morpholinosulfonsäure in Frage. Besonders bevorzugt sind Acylreste bzw. Alkansulfonsäurereste mit 1-4 C-Atomen.

Die Hydroxygruppen in W und $R_4$ können durch Verätherung oder Veresterung funktionell abgewandelt sein, wobei auch die abgewandelte Hydroxygruppe in W α- oder β-ständig sein kann, wobei freie Hydroxygruppen bevorzugt sind.

Benzoyl. Als Alkyl- und Alkenylgruppen $R_5$ kommen gerad- und verzweigtkettige Alkyl- mit 1-6 und Alkenylreste mit 2-6 C-Atomen in Frage, die gegebenenfalls Halogen substituiert sein können Beispielsweise genannt seien Methyl, Äthyl, Propyl, Isobutyl, tert.-Butyl, Pentyl, Hexyl, Butenyl, Isobutenyl, Propenyl, Pentenyl, Hexenyl. Für Halogen als Substituent der Alkyl- und Alkenylgruppen $R_5$ kommen Brom, Chlor und Fluor in Betracht. Bevorzugt sind Chlor und Fluor.

Die Cycloalkylgruppe $R_5$ enthält im Ring 3-6 Kohlenstoffatome. Die Ringe können durch Alkylgruppen mit 1-4 Kohlenstoffatomen substituiert sein. Beispielsweise genannt seien Cyclopropyl, Cyclobutyl, Cyclopentyl, Cyclohexyl, Methyl-cyclohexyl.

Als substituierte bzw. unsubstituierte Arylgruppen $R_5$ kommen in Betracht: Phenyl, 1-Naphthyl und 2-Naphthyl, die jeweils substituiert sein können durch 1-3 Halogenatome, eine Phenylgruppe, 1-3 Alkylgruppen mit jeweils 1-4 C-Atomen, eine Chlormethyl-, Fluormethyl-, Trifluormethyl-, Carboxyl-, Alkoxy- oder Hydroxygruppe. Bevorzugt ist die Substitution in 3- und 4-Stellung am Phenylring zum Beispiel durch Fluor, Chlor, Alkoxy oder Trifluormethyl oder in 4-Stellung durch Hydroxy.

Als heterocyclische Gruppen $R_5$ kommen 2-Furyl, 2-Thienyl, 2-Pyridyl, 3-Pyridyl, 4-Pyridyl, Oxazolyl, Thiazolyl, Pyrimidinyl, Pyridazinyl, Pyrazinyl, 3-Furyl, 3-Thienyl in Frage

Als Alkylengruppe D kommen geradkettige oder verzweigtkettige, ringförmige, gesättigte und ungesättigte Alkylenreste, vorzugsweise gesättigte mit 1-10, insbesondere 1-5 C-Atomen, in Frage, die gegebenenfalls durch Fluoratome substituiert sein können. Beispielsweise seien genannt: Methylen, Fluormethylen, Difluormethylen, Äthylen, 1,2-Propylen, Äthyläthylen, Trimethylen, Tetramethylen, Pentamethylen, 1.1-Difluoräthylen, 1-Fluoräthylen, 1-Methyltetramethylen, 1-Methyl-tri-methylen, 1-Methylen-äthylen, 1-Methylen-tetramethylen, 2-Methyl-trimethylen, 2-Methyltetramethylen, 1,1-Trimethylen-äthylen, 1,2-Methylenäthylen. Wenn eine Doppelbindung vorliegt, befindet sie sich in den Alkylenresten in 2-, 3- oder 4-Stellung.

Zur Salzbildung sind anorganische und organische Basen geeignet, wie sie dem Fachmann zur Bildung physiologisch verträglicher Salze bekannt sind. Beispielsweise seien genannt Alkalihydroxide, wie Natrium- und Kaliumhydroxid, Erdalkalihydroxide, wie Calciumhydroxid, Ammoniak, Amine, wie Äthanolamin, Diäthanolamin, Triäthanolamin, N-Methylglucamin, Morpholin, Tris-(hydroxymethyl)-methylamin usw.

Die Erfindung betrifft außerdem ein Verfahren zur Herstellung der erfindungsgemäßen 9-Halogenprostanderivate der Formel I, dadurch gekennzeichnet, daß man in an sich bekannter Weise eine Verbindung der Formel II

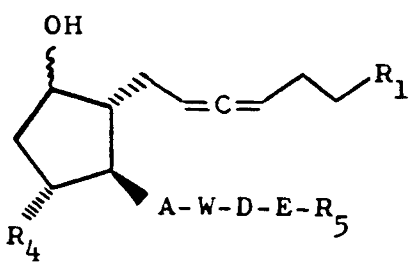

(II),

worin die 9-OH-Gruppe α- oder β-ständig sein kann und

$R_1$ den Rest

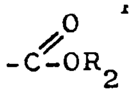

,

mit $R_2$ in der Bedeutung

eines $C_1$-$C_4$-Alkylrestes, welcher gegebenenfalls ein- bis mehrfach durch Fluor, Chlor, Brom, Phenyl, Benzoyl, Dimethylamino, Diethylamino, Methoxy oder Ethoxy substituiert ist, eines $C_5$-$C_6$-

Cycloalkylrestes, oder $R_2$ Phenyl, 1-Naphthyl oder 2-Naphthyl bedeutet, die jeweils substituiert sein können durch 1-3 Halogenatome, eine Phenylgruppe, 1-3 Alkylgruppen mit jeweils 1-4 C-Atomen, eine Chlormethyl-, Fluormethyl-, Trifluormethyl-, Carboxyl-Hydroxy- oder Alkoxygruppe mit 1-4 C-Atomen, oder 2-Furyl, 2-Thienyl, 2-Pyridyl, 3-Pyridyl, 4-Pyridyl, Oxazolyl, Thiazolyl, Pyrimidinyl, Pyridazinyl, 3-Furyl, 3-Thienyl, 2-Tetrazolyl darstellt und A, D, E und $R_5$ die bereits oben angegebenen Bedeutungen haben, nach vorherigem Schutz freier OH-Gruppen in $R_2$, $R_4$ und W mit Tetrachlorkohlenstoff/Triphenylphosphin, Hexachlorethan/Triphenylphosphin oder Diethylaminoschwefeltrifluorid oder nach Umwandlung der 9-Hydroxygruppe in einen 9-Sulfonsäureester mit Tetra-n-butylammoniumfluorid umsetzt und anschließend in beliebiger Reihenfolge geschützte Hydroxygruppen freisetzt und/oder freie Hydroxygruppen verestert, veräthert und/oder Doppelbindungen hydriert und/oder eine veresterte Carboxylgruppe

$$(R_1 = -C\begin{matrix} {}^{\nearrow\!\!\!\nearrow O} \\ {}_{\searrow OR_2}\end{matrix})$$

verseift und/oder eine freie Carboxylgruppe ($R_2$ = H) in ein Amid

$$(R_1 = -C\begin{matrix} {}^{\nearrow\!\!\!\nearrow O} \\ {}_{\searrow NHR_3}\end{matrix})$$

überführt und/oder eine freie oder veresterte Carboxylgruppe

$$(R_1 = -C\begin{matrix} {}^{\nearrow\!\!\!\nearrow O} \\ {}_{\searrow OR_2}\end{matrix})$$

reduziert.

Die Umsetzung der Verbindungen der Formel II zu den Verbindungen der Formel I mit Tetrachlorkohlenstoff und Triphenylphosphin oder Hexachlorethan/Triphenylphosphin erfolgt in einem inerten Lösungsmittel wie beispielsweise Dimethylformamid, Dimethylacetamid, Acetonitril, Methylenchlorid bei Temperaturen zwischen 0 °C und 80 °C, vorzugsweise 20 °C bis 45 °C in Gegenwart einer Base wie beispielsweise Pyridin, Triäthylamin usw.

Die Umsetzung der Verbindungen der Formel II zu den Verbindungen der Formel I mit Hal in der Bedeutung eines Fluoratoms erfolgt mit Diethylaminoschwefeltrifluorid in einem Lösungsmittel wie beispielsweise Dichlormethan bei Temperaturen zwischen -120 °C und 0 °C, vorzugsweise bei -70 °C, gegebenenfalls in Gegenwart einer Base wie beispielsweise Pyridin.

Setzt man einen Alkohol der Formel II mit einer $\beta$-ständigen 9-Hydroxygruppe ein, so erhält man Verbindungen der Formel I mit 9-$\alpha$-ständigem Halogenatom, setzt man einen Alkohol mit einer $\alpha$-ständigen Hydroxygruppe ein, so erhält man Verbindungen mit 9-$\beta$-ständigem Halogenatom.

Die Reduktion zu den Verbindungen der Formel I mit $R_1$ in der Bedeutung einer $-CH_2OH$-Gruppe wird mit einem für die Reduktion von Estern oder Carbonsäuren geeigneten Reduktionsmittel wie beispielsweise Lithiumaluminiumhydrid, Diisobutylaluminiumhydrid usw. durchgeführt. Als Lösungsmittel kommen Diäthyläther, Tetrahydrofuran, Dimethoxyäthan, Toluol usw. in Frage. Die Reduktion wird bei Temperaturen von -30°C bis zur Siedetemperatur des verwendeten Lösungsmittels, vorzugsweise 0°C bis 30°C vorgenommen.

Die Freisetzung der funktionell abgewandelten Hydroxygruppen erfolgt noch bekannten Methoden. Beispielsweise wird die Abspaltung von Hydroxyschutzgruppen, wie beispielsweise des Tetrahydropyranylrestee, in einer wässrigen Lösung einer organischen Säure, wie z.B. Oxalsäure, Essigsäure, Propionsäure u.a., oder in einer wässrigen Lösung einer anorganischen Säure, wie z.B. Salzsäure, durchgeführt. Zur Verbesserung der Löslichkeit wird zweckmässigerweise ein mit Wasser mischbares inertes organisches Lösungsmittel zugesetzt. Geeignete organische Lösungsmittel sind z.B. Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20°C und 80°C durchgeführt.

Die Verseifung der Acylgruppen erfolgt beispielsweise mit Alkali- oder Erdalkali-carbonaten oder

-hydroxyden in einem Alkohol oder in der wässrigen Lösung eines Alkohols. Als Alkohole kommen aliphatische Alkohole in Betracht, wie z.B. Methanol, Äthanol,Butanol usw., vorzugsweise Methanol. Als Alkalicarbonate und -hydroxyde seien Kalium- und Natriumsalze genannt. Bevorzugt sind die Kaliumsalze.

Als Erdalkalicarbonate und -hydroxyde sind beispielsweise geeignet Calciumcarbonat, Calciumhydroxyd und Bariumcarbonat. Die Umsetzung erfolgt bei -10°C bis + 70°C, vorzugsweise bei + 25°C.

Die Einführung der Estergruppe

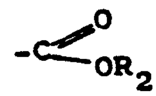

für $R_1$, bei welcher $R_2$ eine Alkylgruppe mit 1-4 C-Atomen darstellt, erfolgt nach den dem Fachmann bekannten Methoden. Die 1-Carboxy-verbindungen werden beispielsweise mit Diazokohlenwasserstoffen in an sich bekannter Weise umgesetzt. Die Veresterung mit Diazokohlenwasserstoffen erfolgt z.B. dadurch, dass man eine Lösung des Diazokohlenwasserstoffes in einem inerten Lösungsmittel, vorzugsweise in Diäthyläther, mit der 1-Carboxyverbindung in dem gleichen oder in einem anderen inerten Lösungsmittel, wie z.B. Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org.Reactions Bd. 8, Seiten 389-394 (1954)].

Die Einführung der Estergruppe

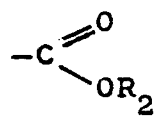

für $R_1$, bei welcher $R_2$ eine substituierte oder unsubstituierte Arylgruppe darstellt, erfolgt nach den dem Fachmann bekannten Methoden. Beispielsweise werden die 1-Carboxyverbindungen mit den entsprechenden Arylhydroxyverbindungen mit Dicyclohexylcarbodiimid in Gegenwart einer geeigneten Base, beispielsweise Pyridin, DMAP, Triäthylamin, in einem inerten Lösungsmittel umgesetzt. Als Lösungsmittel kommen Methylenchlorid, Äthylenchlorid, Chloroform, Essigester, Tetrahydrofuran, vorzugsweise Chloroform in Frage. Die Reaktion wird bei Temperaturen zwischen -30 °C ind +50 °C, vorzugsweise bei 10 °C, durchgeführt.

Die Prostaglandinderivate der Formel I mit $R_2$ in der Bedeutung eines Wasserstoffatoms können mit geeigneten Mengen der entsprechenden anorganischen Base unter Neutralisierung in ein Salz überführt werden. Beispielsweise erhält man beim Lösen der entsprechenden PG-Säuren in Wasser, das die stöchiometrische Menge der Base enthält, nach Abdampfen des Wassers oder nach Zugabe eines mit Wasser mischbaren Lösungsmittels, z.B. Alkohol oder Aceton, das feste anorganische Salz.

Zur Herstellung eines Aminsalzes, die in üblicher Weise erfolgt, wird die PG-Säure z.B. in einem geeigneten Lösungsmittel, beispielsweise Äthanol, Aceton, Diäthyläther, Acetonitril oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die Einführung der Amidgruppe

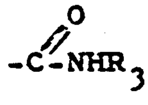

für $R_1$ erfolgt nach den dem Fachmann bekannten Methoden. Die Carbonsäuren der Formel I ($R_2 = H$), werden zunächst in Gegenwart eines tertiären Amins, wie beispielsweise Triäthylamin, mit Chlorameisensäureisobutylester in das gemischte Anhydrid überführt. Die Umsetzung des gemischten Anhydrids mit dem Alkalisalz des entsprechenden Amids oder mit Ammoniak ($R_3 = H$) oder des entsprechenden Amins erfolgt in einen inerten Lösungsmittel oder Lösungsmittelgemisch, wie beispielsweise Tetrahydrofuran, Dimethoxyäthan, Dimethylformamid, Hexamethylphosphorsäuretriamid, bei Temperaturen zwischen -30°Cund +60°C, vorzugsweise bei 0°C bis 30°C.

Eine weitere Möglichkeit für die Einführung der Amidgruppe

$$-\overset{O}{\overset{\|}{C}}-NHR_3$$

für $R_1$ mit $R_3$ in der Bedeutung eines Säurerestes besteht in der Umsetzung einer 1-Carbonsäure der Formel I ($R_2 = H$), in der freie Hydroxygruppen gegebenenfalls intermediär geschützt sind, mit Verbindungen der Formel III

$O = C = N - R_3$ (III),

worin $R_3$ die obenangegebene Bedeutung hat.

Die Umsetzung der Verbindung der Formel I ($R_2 = H$) mit einem Isocyanat der Formel III folgt gegebenenfalls unter Zusatz eines tertiären Amins, wie z.B. Triäthylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diäthyläther, Toluol, bei Temperaturen zwischen -80°C bis 100°C, vorzugsweise bei 0 bis 30°C,vorgenommen werden.

Enthält das Ausgangsprodukt OH-Gruppen im Prostanrest, so werden diese OH-Gruppen auch zur Reaktion gebracht. Werden letztlich Endprodukte gewünscht, die freie Hydroxylgruppen im Prostanrest enthalten, geht man zweckmässigerweise von Ausgangsprodukten aus, in denen diese durch vorzugsweise leicht abspaltbare Äther- oder Acylreste intermediär geschützt sind.

Die als Ausgangsmaterial dienenden Verbindungen der Formel II mit einer 9α-Hydroxygruppe sind entweder bekannt oder können nach dem in DE-OS 26 27 910 angegebenen Verfahren hergestellt werden.

Die Verbindungen der Formel II mit einer 9β-Hydroxygruppe erhält man aus den 9α-Hydroxyverbindungen durch eine Inversionsreaktion, wie sie z.B. in Synthesis, 292-294 (1980) beschrieben wurde.

Im Vergleich zu PGE-Derivaten zeichnen sich die neuen Prostaglandinanaloga durch größere Stabilität aus.

Die neuen Prostaglandinanaloga der Formel I sind wertvolle Pharmaka, da sie bei ähnlichen Wirkungsspektrum eine wesentlich verbesserte (höhere Spezifität) und vor allem wesentlich längere Wirkung aufweisen als die entsprechenden natürlichen Prostaglandine.

Die neuen Prostaglandin-Analoga wirken stark luteolytisch, d.h. zur Auslösung einer Luteolyse benötigt man wesentlich geringere Dosierungen als bei den entsprechenden natürlichen Prostaglandinen.

Auch zur Auslösung von Aborten, insbesondere nach oraler oder intravaginaler Applikation, sind wesentlich geringere Mengen der neuen Prostaglandinanaloga im Vergleich zu den natürlichen Prostaglandinen erforderlich.

Bei der Registrierung der isotonischen Unteruskontraktion an der narkotisierten Ratte und am isolierten Rattenuterus zeigt sich, dass die erfindungsgemässen Substanzen wesentlich wirksamer sind und ihre Wirkungen länger anhalten als bei den natürlichen Prostaglandinen.

Die neuen Prostaglandinderivate sind geeignet, nach einmaliger enteraler oder parenteraler Applikation eine Menstruation zu induzieren oder eine Schwangerschaft zu unterbrechen. Sie eignen sich ferner zur Synchronisation des Sexualzyklus bei weiblichen Säugetieren wie Kaninchen, Rindern, Pferden, Schweinen usw. Ferner eignen sich die erfindungsgemässen Prostaglandin-Derivate zur Cervixdilatation als Vorbereitung für diagnostische oder therapeutische Eingriffe.

Die gute Gewebsspezifität der erfindungsgemässen antifertil wirksamen Substanzen zeigt sich bei der Untersuchung an anderen glattmuskulären Organen, wie beispielsweise am Meerschweinchen-Ileum oder an der isolierten Kaninchen-Trachea, wo eine wesentlich geringere Stimulierung zu beobachten ist als durch die natürlichen Prostaglandine. Die erfindungsgemässen Substanzen wirken auch bronchospasmolytisch. Ausserdem bewirken sie eine Abschwellung der Nasenschleimhaut.

Die erfindungsgemässen Wirkstoffe hemmen die Magensäuresekretion, zeigen einen zytoprotektiven und ulcusheilenden Effekt und wirken damit den unerwünschten Folgen nichtsteroidaler Entzündungshemmstoffe (Prostaglandinsynthese - Inhibitoren) entgegen. Sie wirken ausserdem an der Leber, Niere und auch an der Bauchspeicheldrüse zytoprotektiv.

Einige der Verbindungen wirken blutdrucksenkend, regulierend bei Herzrhytmusstörungen und hemmend auf die Plättchenaggregation mit den sich daraus ergebenden Einsatzmöglichkeiten. Die neuen Prostaglandine können auch in Kombination, z.B. mit β-Blockern, Diuretika, Phospho - diesterasehemmern, Calciumantagonisten und Antigestagenen, verwendet werden.

Die Dosis der Verbindungen ist 1-1500 µg/kg/Tag, wenn sie am menschlichen Patienten verabreicht werden.

Für die medizinische Anwendung können die Wirkstoffe in eine für die Inhalation, für orale, parenterale oder lokale (z.B. vaginale) Applikation geeignete Form überführt werden.

Zur Inhalation werden zweckmässigerweise Ärosollösungen hergestellt.

Für die orale Applikation sind beispielsweise Tabletten, Dragees oder Kapseln geeignet.

Für die parenterale Verabreichung werden sterile, injizierbare, wäßrige oder ölige Lösungen benutzt.

Für die vaginale Applikation sind z.B. Zäpfchen geeignet und üblich.

Die Erfindung betrifft damit auch Arzneimittel auf Basis der Verbindungen der Formel I und üblichen Hilfs- und Trägerstoffe,einschließlich Cyclodextrinclathraten.

Die erfindungsgemäßen Wirkstoffe sollen in Verbindung mit den in der Galenik bekannten und üblichen Hilfsstoffen, z.B. zur Herstellung von Präparaten zur Auslösung eines Abortes, zur Zyklussteuerung, zur Einleitung einer Geburt, zur Behandlung der Hypertonie oder zur Behandlung von gastrointestinalen Störungen, wie z.B. zur Abheilung von Magen- und Zwölffingerdarmgeschwüren, dienen. Für diesen Zweck aber auch für die übrigen Anwendungen können die Präparate 0,01 - 100 mg der aktiven Verbindung enthalten.

Die folgenden Beispiele sollen die Erfindung näher erläutern, ohne daß damit eine Begrenzung vorgenommen wird.

Beispiel 1

(9R,11R,15R)-9-Chlor-11,15-dihydroxy-16,16-dimethyl-prosta-4,5,13-trans-triensäuremethylester

Zu einer Lösung aus 1,80 g (9S,11R,15R)-16,16-Dimethyl-9-hydroxy-11,15-bis(tetrahydropyran-2-yloxy)-prosta-4,5,13-trans-triensäuremethylester und 3,37 g Triphenylphosphin in 60 ml 1,2-Dichlorethan tropft man bei 0°C eine Lösung von 3,20 g Hexachlorethan und 3,80 ml Triethylamin in 60 ml 1,2 Dichloräthan. Man rührt 1 Stunde bei 20°C, verdünnt dann mit 200 ml Dichlormethan, schüttelt nacheinander mit Natriumhydrogencarbonatlösung und Sole, trocknet über $M_gSO_4$ und dampft im Vakuum ein. Der Rückstand wird an Kieselgel mit Toluol/Ethylacetat (95:5) chromatographiert. Man erhält 1,05 g öligen (9R,11R,15R)-9-Chlor-16,16-dimethyl-11,15-bis(tetrahydropyran-2-yloxy)-prosta-4,5,13-trans-triensäuremethylester. Zur Abspaltung der Schutzgruppen rührt man den erhaltenen Ester mit 15 ml einer Mischung aus Essigsäure/Wasser/Tetrahydrofuran (65/35/10) 24 Stunden bei 20°C. Man verdünnt dann mit Eiswasser, versetzt bis zum Neutralpunkt mit verdünnter Natronlauge und extrahiert mehrmals mit Dichlormethan. Die vereinigten Extrakte werden mit Sole geschüttelt, über Magnesiumsulfat getrocknet und im Vakuum eingedampft. Zur Reinigung chromatographiert man an Kieselgel mit Hexan/10-40% Ethylacetat und erhält 580 mg der Titelverbindung als Öl.

IR: 3600, 3410, 2958, 1958, 1732, 1135, 1020, 976/cm.

Der als Ausgangsmaterial verwendete 9α-Alkohol wird wie folgt erhalten:

1a)

(6RS,9S,11R,15R )-6,9-Dihydroxy-16,16-dimethyl-11,15-bis (tetrahydropyran-2-yloxy)-prost-4-in-13-trans-ensäuremethylester

Man löst 7,29g Diisopropylamin in 100 ml Ether mit 12,90 g Hexamethylphosphorsäuretriamid, kühlt auf -20°C ab und tropft unter Argon 48 ml einer 1,5 M etherischen Lösung von Methyllithium zu. Anschließend kühlt man auf -70°C ab und tropft 3,53 g 4-Pentinsäure gelöst in 70ml Ether zu. man rührt weitere 2 Stunden bei 20°C und tropft dann eine Lösung von 2,80 g (2RS,3aR,4R,5R,6aS)-4-[(E)-(3R)-4,4-dimethyl-3-(tetrahydropyran-2-yloxy)-1-octenyl]-5-(tetrahydropyran-2-yloxy)-perhydrocyclopenta[b]-furan-2-ol in 60 ml Ether zu. Man rührt 48 Stunden bei 20°C, verdünnt mit Wasser und säuert mit Zitronensäure auf pH k an. Man extrahiert mehrmals mit Dichlormethan, wäscht mit Sole, trocknet über Magnesiumsulfat und dampft im Vakuum ein. Der Rückstand wird 15 Minuten mit überschüssigem etherischen Diazomethan behandelt und die Lösung zur Trockne gedampft. Der ölige Rückstand wird an Kieselgel mit Hexan/Ethylacetat (1:1) chromatographiert. Man erhält 2,15 g der Titelverbindung als Öl.

IR: 3600, 2955, 2130, 1735, 1153, 1020, 980/cm.

1b)

(6RS,9S,11R,15R)-6,9-Diacetoxy-16,16-dimethyl-11,15-bis(tetrahydropyran-2-yloxy)-prost-4-in-13-trans-ensäuremethylester

Zu einer Lösung von 2,10 g (6RS,9S,11R,15R)-6,9-Dihydroxy-16,16-dimethyl-11,15-bis(tetrahydropyran-2-yloxy)-prost-4-in-13-trans-ensäuremethylester in 15 ml Pyridin gibt man 4 ml Essigsäureanhydrid und läßt 16 Stunden bei 20°Cstehen. Man engt dann im Vakuum ein, verdünnt mit Äther, wäscht mit Wasser,

trocknet über Magnesiumsulfat und dampft im Vakuum ein. Zur Reinigung filtriert man mit Hexan/Ethylacetat (7:5) über Kieselgel und erhält 2,19 g der Titelverbindung als Öl.
IR: 2958, 2125, 1738,1252, 1023, 976/cm.
1c)
(9S,11R,15R)-9-Acetoxy-16,16-dimethyl-11,15-bis-(tetrahydropyran-2-yloxy)-prosta-4,5,13-trans-triensäuremethylester
Zu einer auf -10°C gekühlten Suspension von 3,02 g Kupfer (I)jodid in 50 ml Ether tropft man unter Rühren 23,2 ml einer 1.5 M Lösung von Methyllithium in Ether. Man kühlt dann auf -75°C ab und tropft 50 ml einer etherischen Lösung von 2,10 g (6RS,9S,11R,15R)-6,9-Diacetoxy-16,16-dimethyl-11,15-bis-(tetrahydropyran-2-yloxy)-prost-4-in-13-transensäuremethylester innerhalb von 15 Minuten zu. Man rührt 5 Stunden bei -75°C, verdünnt dann mit Ammoniumchloridlösung, rührt 1 Stunde bei 20°C und extrahiert mit Äther. Den Extrakt wäscht man mit Sole, trocknet über Magnesiumsulfat und dampft im Vakuum ein. Den Rückstand chromatographiert man an Kieselgel mit Hexan/20-70% Ethylacetat und erhält 1,10 g der Titelverbindung als Öl.
IR: 2951, 1977, 1738. 1248, 1021, 978/cm.
1d)
(9S,11R,15R)-16,16-Dimethyl-9-hydroxy-11,15-bis-(tetrahydropyran-2-yloxy)-prosta-4,5,13-trans-triensäuremethylester
Zu einer Lösung von 1,05 g (9S,11R,15R)-9-Acetoxy-16,16-dimethyl-11,15-bis-(tetrahydropyran-2-yloxy)-prosta-4,5,13-trans-triensäuremethylester in 20 ml Methanol gibt man 300 mg wasserfreies Kaliumcarbonat und rührt 3 Stunden bei Raumtemperatur.Man engt bei 30°C im Vakuum ein, verdünnt mit Wasser und extrahiert mehrmals mit Dichlormethan. Die vereinigten Extrakte werden mit Sole geschüttelt, über Magnesiumsulfat getrocknet und im Vakuum eingedampft. Man erhält 920 mg der Titelverbindung als Öl.
IR: 3600, 2945, 1980. 1736, 1021, 976/cm.

## Beispiel 2

(9S,11R,15R)-9-Chlor-11,15-dihydroxy-16,16-dimethyl-prosta-4,5,13-trans-triensäuremethylester

In Analogie zu Beispiel 1 erhält man aus 500 mg (9R,11R, 15R )-16,16-Dimethyl-9-hydroxy-11,15-bis-(tetrahydropyran-2-yloxy)-prosta-4,5,13-trans-triensäuremethylester 145 mg der Titelverbindung als Öl.
IR: 3600, 3405, 2956, 1977, 1735, 1135, 1021, 976/cm.

Der als Ausgangsmaterial verwendete 9β-Alkohol wird wie folgt hergestellt:
2a)
(9R,11R,15R )-16,16-Dimethyl-9-hydroxy-11,15-bis-(tetrahydropyran-2-yloxy)-prosta-4,5,13-trans-triensäuremethylester
Zu einer Lösung von 1,69 g (9S,11R,15R)-16,16-Dimethyl-9-hydroxy-11,15-bis-(tetrahydropyran-2-yloxy)-prosta-4,5,13-trans-triensäuremethylester in 25 ml Pyridin gibt man bei 0° 1,15 g p-Toluolsulfonsäurechlorid. Nach 1 Stunde entfernt man das Eisbad und läßt 48 Stunden bei 20°C stehen. Man kühlt dann wieder auf 0°, versetzt mit o,1 ml Wasser und rührt 1 Stunde. Zur Aufarbeitung verdünnt man mit eiskaltem Äther, schüttelt nacheinander mit eiskalter 10%iger Schwefelsäure, Natriumhydrogencarbonatlösung und Sole, trocknet über Magnesiumsulfat und dampft in Vakuum ein. Man erhält 2,30 g öliges 9-Tosylat, das man in 80 ml Dimethylsulfoxid löst, mit 6 g Kaliumnitrit versetzt und 3 Stunden auf 80°C erhitzt. Man verdünnt dann mit Wasser, extrahiert mit Ether, wäscht den Extrakt mit Sole, trocknet über Magnesiumsulfat und dampft im Vakuum ein. Den Rückstand reinigt man durch Chromatographie an Kieselgel mit Hexan/Ethylacetat (20-50%) und erhält 1,01 g der Titelverbindung als Öl.
IR: 3600, 3410, 1978, 1735, 1181, 1025, 972/cm.

## Beispiel 3

(9R,11R,15R)-9-Chlor-11,15-dihydroxy-16,16-dimethyl-prosta-4,5,13-trans-triensäure

Zu einer Lösung von 100 mg (9R,11R,15R)-9-Chlor-11,15-dihydroxy-16,16-dimethyl-prosta-4,5,13-trans-triensäuremethylester in 10 ml Methanol gibt man 150 mg Kaliumhydroxid gelöst in 1 ml Wasser und läßt 5 Stunden bei 20°C stehen. Nach Einengen im Vakuum verdünnt man mit Wasser, säuert mit Zitronensäure auf pH 4 an und extrahiert mit Ethylacetat. Den Extrakt wäscht man mit Sole, trocknet über Magnesiumsulfat und dampft im Vakuum ein. Man erhält 85 mg der Titelverbindung als Öl.
IR: 3600, 3420, 2955, 1976, 1712, 1178, 1022, 976/cm.

Beispiel 4

(9R,11R,15S,16RS)-9-Chlor-11,15-dihydroxy-16-methyl-prosta-4,5,13-trans-triensäuremethylester

In Analogie zu Beispiel 1 erhält man aus 1,25 g (9S,11R,15S, 16RS)-9-Hydroxy-16-methyl-11,15-bis-(tetrahydropyran-2-yloxy)-prosta-4,5,13-trans-triensäure-methylester 410 mg der Titelverbindung als Öl.
IR: 3600, 3405, 2958, 2885, 1976, 1732, 1021, 976/cm.

Das Ausgangsmaterial für die Herstellung der Titelverbindung erhält man gemäß Beispiel 1a aus (2RS,3aR,4R,5R,6aS)-4-[(E)-(3S,4RS)-4-methyl-3-(tetrahydropyran-2-yloxy)-1-octenyl]-5-(tetrahydropyran-2-yloxy)-perhydrocyclopenta[b]furan-2-ol.

Beispiel 5

(9R,11R,15S,16RS)-9-Chlor-11,15-dihydroxy-16-methyl-prosta-4,5,13-trans-triensäure

In Analogie zu Beispiel 3 erhält man aus (9R,11R,15S,16RS)-9-Chlor-11,15-dihydroxy-16-methyl-prosta-4,5,13-trans-triensäuremethylester die Titelverbindung als Öl.
IR: 3600, 3420, 2948, 1978, 1710, 1021, 978/cm.

Beispiel 6

(9R,11R,15R)-9-Chlor-11,15-dihydroxy-16-phenoxy-17,18,19, 20-tetranor-prosta-4,5,13-trans-triensäuremethylester

In Analogie zu Beispiel 1 erhält man aus 950 mg (9S,11R, 15R)-9-Hydroxy-16-phenoxy-11,15-bis-(tetrahydropyran-2-yloxy)-17,18,19,20-tetranor-prosta-4,5,13-trans-triensäuremethylester 310 mg der Titelverbindung als farbloses Öl.
IR: 3600, 3402, 2954, 2888, 1978, 1732, 1600, 1585, 1021, 978/cm.

Das Ausgangsmaterial für die Herstellung der Titelverbindung erhält man aus (2RS, 3aR,4R,5R,6aS)-4-[-(E)-(3R)-4-Phenoxy-3-(tetrahydopyran-2-yloxy)-1-butenyl]-5-(tetrahydropyran-2-yloxy)-perhydrocyclopenta-[b]furan-2-ol gemäß Beispiel 1a.

Beispiel 7

(9R,11R,15R)-9-Chlor-11,15-dihydroxy-16-phenoxy-17,18,19,20-tetranor-prosta-4,5,13-trans-triensäure

In Analogie zu Beispiel 3 erhält man aus (9R,11R,15R)-9-Chlor-11,15-dihydroxy-16-phenoxy-17,18,19,20-tetranor-prosta-4,5,13-trans-triensäuremethylester die Titelverbindung als Öl.
IR: 3600, 3420, 2960, 2885, 1976, 1711, 1600, 1588, 1022, 977/cm.

Beispiel 8

(9R,11R,15R)-9-Chlor-11,15-dihydroxy-16,16-trimethylen-prosta-4,5,13-trans-triensäuremethylester

In Analogie zu Beispiel 1 erhält man aus 1,20 g (9S,11R, 15R)-9-Hydroxy-11,15-bis-(tetrahydropyran-2-yloxy)-16,16-trimethylen-prosta-4,5,13-trans-triensäuremethylester 390 mg der Titelverbindung als Öl.
IR: 3600, 3405, 2948, 2882, 1975, 1735, 1021, 976/cm.

Das Ausgangsmaterial für die Herstellung der Titelverbindung erhält man aus (2RS,3aR,4R,5R,6aS)-4-[-(E)-(3R)-3-(tetrahydropyran-2-yloxy)-4,4-trimethylen-1-octenyl]-5-(tetrahydropyran-2-yloxy)-perhydrocyclopenta[b]furan-2-ol.

Beispiel 9

(9R,11R,15R)-9-Chlor-11,15-dihydroxy-16,16-trimethylen-prosta-4,5,13-trans-triensäure

In Änalogie zu Beispiel 3 erhält man aus (9R,11R,15R)-9-Chlor-11,15-dihydroxy-16,16-trimethylen-prosta-4,5,13-trans-triensäuremethylester die Titelverbindung als Öl.
IR: 3600, 3420, 2952, 2884, 1976, 1712, 1022, 976/cm.

Beispiel 10

(9R,11R,15S,16RS)-9-Chlor-11,15-dihydroxy-16-methyl-prosta-4,5,13-trans-trien-18-insäuremethylester

In Analogie zu Beispiel 1 erhält man aus 985 mg (9S,11R, 15S,16RS)-9-Hydroxy-16-methyl-11,15-bis-(tetrahydropyran-2-yloxy)-prosta-4,5,13-trans-trien-18-insäuremethylester 320 mg der Titelverbindung als Öl.
IR: 3600, 3405, 2050, 1978, 1734, 1021, 974/cm.

Das Ausgangsmaterial für die Herstellung der Titelverbindung erhält man gemäß Beispiel 1a aus (2RS,3aR,4R,5R,6aS)-4-[(E)-(3S,4RS)-4-methyl-3-(tetrahydropyran-2-yloxy)-1-octen-6-inyl]-5-(tetrahydropyran-2-yloxy)-perhydrocyclopenta[b]furan-2-ol.

Beispiel 11

(9R,11R,15S,16RS)-9-Chlor-11,15-dihydroxy-16-methyl-prosta-4,5,13-trans-trien-18-insäure

In Analogie zu Beispiel 3 erhält man aus(9R,11R,15S,16RS)-9-Chlor-11,15-dihydroxy-16-methyl-prosta-4,5,13-transtrien-18-insäuremethylester die Titelverbindung als Öl.
IR: 3600, 3415,2955, 2884, 1978, 1710, 1022, 974/cm.

Beispiel 12

(9R,11R,15S,16RS)-9-Chlor-11,15-dihydroxy-16,20-dimethyl-prosta-4,5,13-trans-trien-18-insäuremethylester

In Analogie zu Beispiel 1 erhält man aus 1,20 g (9S,11R, 15S,16RS)-16,20-Dimethyl-9-hydroxy-11,15-bis-(tetrahydropyran-2-yloxy)-prosta-4,5,13-trans-trien-18-insäuremethylester 380 mg der Titelverbindung als Öl.
IR: 3600, 3400, 2955, 1978, 1732, 1022, 976/cm.

Das Ausgangsmaterial für die Herstellung der Titelverbindung erhält man gemäß Beispiel 1a aus (2RS,3aR,4R,5R,6aS)-4-[(E)-(3S,4RS)-4-methyl-3-(tetrahydropyran-2-yloxy)-1-nonen-6-inyl]-5-(tetrahydropyran-2-yloxy)-perhydrocyclopenta[b]furan-2-ol.

Beispiel 13

(9R,11R,15S,16RS)-9-Chlor-11,15-dihydroxy-16,20-dimethyl-prosta-4,5,13-trans-trien-18-insäure

In Analogie zu Beispiel 3 erhält man aus (9R,11R,15S,16RS)-9-Chlor-11,15-dihydroxy-16,20-dimethyl-prosta-4,5,13-transtrien-18-insäuremethylester die Titelverbindung als Öl.
IR: 3600, 3409, 2952, 2880, 1978, 1711, 1023, 976/cm.

Beispiel 14

(9R,11R,15R)-11,15-Dihydroxy-16,16-dimethyl-9-fluor-prosta-4,5,13-trans-triensäure

Zu einer Lösung von 1,25 g (9S,11R,15R)-16,16-Dimethyl-9-hydroxy-11,15-bis-(tetrahydropyran-2-yloxy)-prosta-4,5,13-trans-triensäuremethylester in 20 ml Dichlormethan und 0,5 ml Pyridin gibt man bei -70°C 0,3 ml Diethylaminoschwefeltrifluorid (DAST) und nach 15 Minuten nochmals 0,1 ml DAST. Nach weiteren 15 Minuten wird mit 50 ml 5%iger Natriumhydrogencarbonatlösung versetzt, das Kältebad entfernt, 10 Minuten bei 20° kräftig gerührt, dann mit Dichlormethan extrahiert, der Extrakt mit Sole gewaschen, über Magnesiumsulfat getrocknet und im Vakuum eingedampft. Den Rückstand rührt man 24 Stunden mit 20 ml einer Mischung aus Essigsäure-Wasser-Tetrahydrofuran (65/35/10), dampft im Vakuum ein und reinigt das Rohprodukt durch Chromatographie an Kieselgel mit Hexan/Diethylether (1:1). Man erhält 410 mg (9R,11R,15R)-11,15-Dihydroxy-16,16-dimethyl-9-fluor-prosta-4,5,13-trans-triensäuremethylester als Öl.
Zur Verseifung des Esters löst man in 20ml Methanol, versetzt mit 500 mg Kaliumhydroxid gelöst in 2 ml Wasser und läßt 4 Stunden bei 20° stehen. Anschließend engt man in Vakuum ein, verdünnt mit Wasser, säuert mit Zitronensäure auf pH4 an und extrahiert mit Dichlormethan. Den Extrakt wäscht man mit Sole, trocknet über Magnesiumsulfat und dampft im Vakuum ein. Man erhält 370 mg der Titelverbindung als Öl.
IR: 3600, 3420, 2948, 2878, 1976, 1710, 1022, 978/cm.

Beispiel 15

(9R,11R,15R)-11,15-Dihydroxy-9-fluor-16-phenoxy-17,18,19, 20-tetranor-prosta-4,5,13-trans-triensäure

In Analogie zu Beispiel 14 erhält man aus 1,15 g (9S,11R, 15R)-9-Hydroxy-16-phenoxy-11,15-bis-(tetrahydropyran-2-yloxy)-17,18,19,20-tetranor-prosta-4,5,13-trans-triensäure-methylester 335 mg der Titelverbindung als Öl.
IR: 3600, 3420, 2955, 2889, 1978, 1708, 1601, 1588, lo23, 976/cm.

Beispiel 16

(9R,11R,15S,16RS)-11,15-Dihydroxy-16,20-dimethyl-9-fluor-prosta-4,5,13-trans-trien-18-insäure

In Analogie zu Beispiel 14 erhält man aus 810 mg (9S,11R, 15S,16RS)-16,20-Dimethyl-9-hydroxy-11,15-bis-(tetrahydropyran-2-yloxy)-prosta-4,5,13-trans-trien-18-insäuremethylester 235 mg der Titelverbindung als Öl.
IR: 3600, 3418, 2949, 2822, 1976, 1710, 1023, 978/cm.

Beispiel 17

(9R,11R,15R)-11,15-Dihydroxy-9-fluor-16-phenoxy-17,18,19,20-tetranor-prosta-4,5,13-trans-triensäure-phenacylester

Man löst 210 mg (9R,11R,15R)-11,15-Dihydroxy-9-fluor-16-phenoxy-17,18,19,20-tetranor-prosta-4,5,13-trans-triensäure in 10 ml Aceton, versetzt mit 139 mg ω-Bromacetophenon und 1,5 ml Triätrylamin und rührt übel Nacht bei 20°C. Man verdünnt mit Äther,schüttelt nacheinander mit Wasser und Sole, trocknet über Magnesiumsulfat und dampft im Vakuum ein. Den Rückstand reinigt man durch Chromatographie an Kieselgel mit Dichlormethan/10% Aceton und erhält 195 mg der Titelverbindung als Öl.
IR: 3600, 3010, 2948, 1978, 1708(breit), 1600, 1588, 1139, 1022, 974/cm.

## Patentansprüche

**1.** 9-Halogen-prostanderivate der Formel I

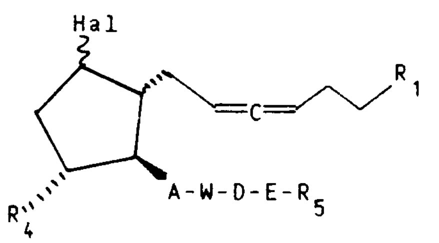

(I),

worin

Hai ein α- oder β-standiges Chlor- oder Fluoratom,

$R_1$ den Rest $CH_2OH$ oder

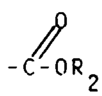

mit $R_2$ in der Bedeutung eines Wasserstoffatoms, eines $C_1$-$C_4$-Alkylrestes, welcher gegebenenfalls ein- bis mehrfach durch Fluor, Chlor, Brom, Phenyl, Benzoyl, Dimethylamino, Diethylamino, Methoxy oder Ethoxy substituiert ist, eines $C_5$-$C_6$-Cycloalkylrestes, oder $R_2$ Phenyl, 1-Naphthyl oder 2-Naphthyl bedeutet,
die jeweils substituiert sein können

durch 1-3 Halogenatome, eine Phenylgruppe, 1-3 Alkylgruppen mit jeweils 1-4 C-Atomen, eine Chlormethyl-, Fluormethyl-, Trifluormethyl-, Carboxyl-Hydroxy- oder Alkoxygruppe mit 1-4 C-Atomen, oder 2-Furyl, 2-Thienyl, 2-Pyridyl, 3-Pyridyl, 4-Pyridyl, Oxazolyl, Thiazolyl, Pyrimidinyl, Pyridazinyl, 3-Furyl, 3-Thienyl, 2-Tetrazolyl oder $R_1$ den Rest

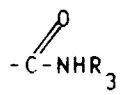

mit $R_3$ in der Bedeutung eines organischen Carbonsäure- oder Sulfonsäurerestes mit 1-15 C-Atomen oder des Restes $R_2$ und

A eine $-CH_2-CH_2-$, eine trans-CH = CH- oder eine -C≡C-Gruppe,

W eine freie oder durch Tetrahydropyranyl, Tetrahydrofuranyl, $\alpha$-Äthoxyäthyl, Trimethylsilyl, Dimethyl-tert.-.butyl-silyl, Tribenzylsilyl, Acetyl, Propionyl, Butyryl und Benzoyl verätherte oder veresterte Hydroxymethylengruppe oder eine freie oder durch Tetrahydropyranyl, Tetrahydrofuranyl, $\alpha$-Äthoxyäthyl, Trimethylsilyl, Dimethyl-tert.-butyl-silyl, Acetyl, Propionyl, Butyryl und Benzoyl verätherte oder veresterte

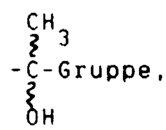

wobei die OH-Gruppe $\alpha$- oder $\beta$-standig sein kann.

D und E gemeinsam eine direkte Bindung oder

D eine geradkettige, verzweigte oder ringförmige Alkylengruppe mit 1-10 C-Atomen, die gegebenenfalls durch Fluoratome substituiert ist, und

E ein Sauerstoff- oder Schwefelatom, eine direkte Bindung, eine -C≡C-Bindung oder eine $-CR_6 = CR_7$-Gruppe darstellt, wobei $R_6$ und $R_7$ sich unterscheiden und ein Wasserstoffatom, ein Chloratom oder eine $C_1$-$C_4$-Alkylgruppe bedeuten,

$R_4$ eine freie oder durch Tetrahydropyranyl, Tetrahydrofuranyl, $\alpha$-Äthoxyäthyl, Trimethylsilyl, Dimethyl-tert.-.butyl-silyl, Tribenzylsilyl, Acetyl, Propionyl, Butyryl und Benzoyl verätherte oder veresterte Hydroxygruppe,

$R_5$ ein Wasserstoffatom, eine Alkyl- oder Alkenylgruppe mit bis zu 6 C-Atomen, eine Halogen-substituierte Alkyl- oder Alkenylgruppe mit bis zu 6 C-Atomen, eine $C_3$-$C_6$-Cycloalkylgruppe, Phenyl, 1-Naphthyl und 2-Naphthyl, die je-weils substituiert sein können durch 1-3 Halogenatome, eine Phenylgruppe, 1-3 Alkylgruppen mit jeweils 1-4 C-Atomen, eine Chlormethyl-, Fluormethyl-, Trifluormethyl-, Carboxyl-, Hydroxy- oder Alkoxygruppe mit 1-4 C-Atomen oder $R_5$ 2-Furyl, 2-Thienyl, 2-Pyridyl, 3-Pyridyl, 4-Pyridyl, Oxazolyl, Thiazolyl, Pyrimidinyl, Pyridazinyl, Pyrazinyl, 3-Furyl, 3-Thienyl, bedeuten und falls $R_2$ die Bedeutung eines Wasserstoffatoms hat, deren Salze mit physiologisch verträglichen Basen.

2. Verfahren zur Herstellung der 9-Halogen-prostanderivate der Formel I, dadurch gekennzeichnet, daß man in an sich bekannter Weise eine Verbindung der Formel II

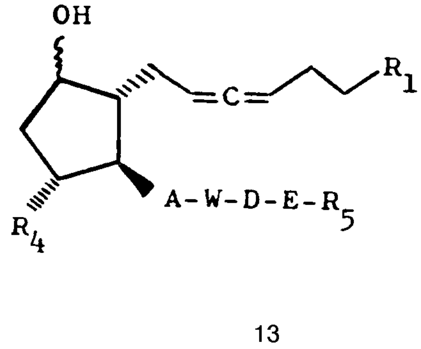

(II),

worin die 9-OH-Gruppe α- oder β-ständig sein kann und
$R_1$ den Rest

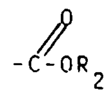

mit $R_2$ in der Bedeutung eines $C_1$-$C_4$-Alkylrestes, welcher gegebenenfalls ein- bis mehrfach durch Fluor, Chlor, Brom, Phenyl, Benzoyl, Dimethylamino, Diethylamino, Methoxy oder Ethoxy substituiert ist, eines $C_5$-$C_6$-Cycloalkylrestes, oder $R_2$ Phenyl, 1-Naphthyl oder 2-Naphthyl bedeutet,
die jeweils substituiert sein können
durch 1-3 Halogenatome, eine Phenylgruppe, 1-3 Alkylgruppen mit jeweils 1-4 C-Atomen, eine Chlormethyl-, Fluormethyl-, Trifluormethyl-, Hydroxy- oder Alkoxygruppe mit 1-4 C-Atomen, oder 2-Furyl, 2-Thienyl, 2-Pyridyl, 3-Pyridyl, 4-Pyridyl, Oxazolyl, Thiazolyl, Pyrimidinyl, Pyridazinyl, 3-Furyl, 3-Thienyl, 2-Tetrazolyl oder
$R_1$ den Rest

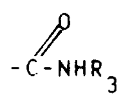

mit $R_3$ in der Bedeutung eines organischen Carbonsäure- oder Sulfonsaurerestes mit 1-15 C-Atomen oder des Restes $R_2$ und A, D, E und $R_5$ die bereits oben angegebenen Bedeutungen haben, nach vorherigem Schutz freier OH-Gruppen in $R_2$,$R_4$ und W mit Tetrachlorkohlenstoff/Triphenylphosphin, Hexachlorethan/Triphenylphosphin oder Diethylaminoschwefeltrifluorid oder nach Umwandlung der 9-Hydroxygruppe in einen 9-Sulfonsäureester mit Tetra-n-butylammoniumfluorid unsetzt und anschließend in beliebiger Reihenfolge geschützte Hydroxygruppen freisetzt und/oder freie Hydroxygruppen verestert, veräthert und/oder Doppelbindungen hydriert und/oder eine veresterte Carboxylgruppe

$$(R_1 = -C(=O)OR_2)$$

verseift und/oder eine freie Carboxylgruppe ($R_2$ = H) in ein Amid

$$(R_1 = -C(=O)NHR_3)$$

überführt und/oder eine freie oder veresterte Carboxylgruppe

$$(R_1 = -C(=O)OR_2)$$

reduziert.

**3.** Arzneimittel, bestehend aus einer oder mehreren Verbindungen des Anspruchs 1 und üblichen Hilfs- und Trägerstoffen.

## Claims

**1.** 9-Halo-prostane derivatives of formula I

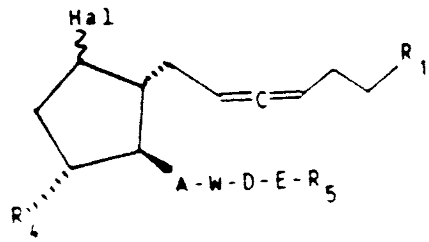

(I)

wherein

Hal represents an $\alpha$- or $\beta$-configured chlorine or fluorine atom,

$R_1$ represents the radical $CH_2OH$ or

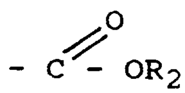

in which $R_2$ represents a hydrogen atom, a $C_1$-$C_4$alkyl radical that is optionally mono- or polysubstituted by fluorine, chlorine, bromine, phenyl, benzoyl, dimethylamino, diethylamino, methoxy or by ethoxy, or a $C_5$-$C_6$cycloalkyl radical, or $R_2$ represents phenyl, 1-naphthyl or 2-naphthyl, each of which may be substituted by from 1 to 3 halogen atoms, by a phenyl group, by from 1 to 3 alkyl groups each having from 1 to 4 carbon atoms, by a chloromethyl, fluoromethyl, trifluoromethyl, carboxy or hydroxy group or by an alkoxy group having from 1 to 4 carbon atoms, or $R_2$ represents 2-furyl, 2-thienyl, 2-pyridyl, 3-pyridyl, 4-pyridyl, oxazolyl, thiazolyl, pyrimidinyl, pyridazinyl, 3-furyl, 3-thienyl or 2-tetrazolyl, or

$R_1$ represents the radical

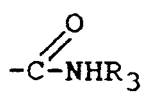

in which $R_3$ represents an organic carboxylic acid or sulphonic acid radical having from 1 to 15 carbon atoms, or represents the radical $R_2$, and

A represents a $-CH_2-CH_2-$, a trans-$CH=CH-$ or a $-C\equiv C-$group,

W represents a hydroxymethylene group that is free or is etherified or esterified by tetrahydropyranyl, tetrahydrofuranyl, $\alpha$-ethoxyethyl, trimethylsilyl, dimethyl-tert.-butyl-silyl, tribenzylsilyl, acetyl, propionyl, butyryl or by benzoyl, or represents a

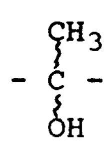

group,

in which the OH group may be $\alpha$- or $\beta$-configured, that is free or is etherified or esterified by tetrahydropyranyl, tetrahydrofuranyl, $\alpha$-ethoxyethyl, trimethylsilyl, dimethyl-tert.-butyl-silyl, acetyl, propionyl, butyryl or by benzoyl,

D and E together represent a direct bond or

D represents a straight-chain, branched or cyclic alkylene group having from 1 to 10 carbon atoms, which is optionally substituted by fluorine atoms, and

E represents an oxygen or sulphur atom, a direct bond, a $-C\equiv C-$ bond or a $-CR_6=CR_7-$ group wherein $R_6$ and $R_7$ are different from one another and each represents a hydrogen atom, a chlorine atom or a $C_1$-$C_4$alkyl group,

$R_4$ represents a hydroxy group that is free or is etherified or esterified by tetrahydropyranyl, tetrahydrofuranyl, $\alpha$-ethoxyethyl, trimethylsilyl, dimethyl-tert.-butyl-silyl, acetyl, propionyl, butyryl or by benzoyl,

$R_5$ represents a hydrogen atom, an alkyl or alkenyl group having up to 6 carbon atoms, a halo-substituted alkyl or alkenyl group having up to 6 carbon atoms, a $C_3$-$C_6$ cycloalkyl group, phenyl, 1-naphthyl or 2-naphthyl, each of which may be substituted by from 1 to 3 halogen atoms, by a phenyl group, by from 1 to 3 alkyl groups each having from 1 to 4 carbon atoms, or by a chloromethyl, fluoromethyl, trifluoromethyl, carboxy or hydroxy group or by an alkoxy group having from 1 to 4 carbon atoms, or $R_5$ represents 2-furyl, 2-thienyl, 2-pyridyl, 3-pyridyl, 4-pyridyl, oxazolyl, thiazolyl, pyrimidinyl, pyridazinyl, pyrazinyl, 3-furyl or 3-thienyl,

and, if $R_2$ represents a hydrogen atom, the salts thereof with physiologically tolerable bases.

**2.** A process for the preparation of the 9-halo-prostane derivatives of formula I, characterised in that, in a manner known per se, a compound of formula II

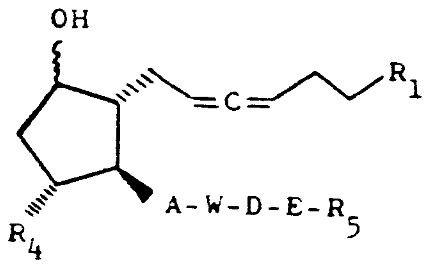

(II)

wherein the 9-OH group may be $\alpha$- or $\beta$-configured and

$R_1$ represents the radical

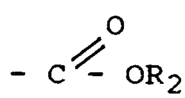

in which $R_2$ represents a $C_1$-$C_4$ alkyl radical that is optionally mono- or poly-substituted by fluorine, chlorine, bromine, phenyl, benzoyl, dimethylamino, diethylamino, methoxy or by ethoxy, or a $C_5$-$C_6$ cycloalkyl radical, or $R_2$ represents phenyl, 1-naphthyl or 2-naphthyl, each of which may be substituted by from 1 to 3 halogen atoms, by a phenyl group, by from 1 to 3 alkyl groups each having from 1 to 4 carbon atoms, by a chloromethyl, fluoromethyl, trifluoromethyl or hydroxy group or by an alkoxy group having from 1 to 4 carbon atoms, or $R_2$ represents 2-furyl, 2-thienyl, 2-pyridyl, 3-pyridyl, 4-pyridyl, oxazolyl, thiazolyl, pyrimidinyl, pyridazinyl, 3-furyl, 3-thienyl, 2-tetrazolyl, or $R_1$ represents the radical

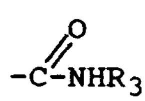

in which $R_3$ represents an organic carboxylic acid or sulphonic acid radical having from 1 to 15 carbon atoms, or represents the radical $R_2$, and

A, D, E and $R_5$ have the meanings already given,

after previous protection of free OH groups in $R_2$, $R_4$ and W, is reacted with carbon tetrachloride/triphenylphosphine, hexachloroethane/triphenylphosphine or diethylaminosulphur trifluoride or, after conversion of the 9-hydroxy group into a 9-sulphonic acid ester, with tetra-n-butylammonium fluoride, and then, in any sequence, protected hydroxy groups are freed and/or free hydroxy groups are esterified or etherified and/or double bonds are hydrogenated and/or an esterified carboxy group

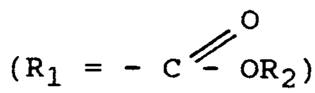

is hydrolysed and/or a free carboxy group ($R_2$ = H) is converted into an amide

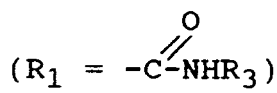

and/or a free or esterified carboxy group

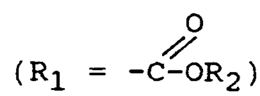

is reduced.

**3.** Medicament, consisting of one or more compounds of claim 1 and customary excipients and carriers.

**Revendications**

**1.** Dérivés de 9-halogéno-prostanes de formule I ci-dessous :

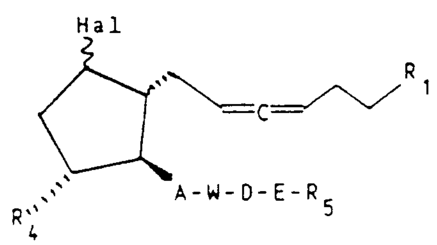

(I)

dans laquelle

Hal désigne un atome de chlore ou de fluor en position $\alpha$ ou $\beta$,

$R_1$ un radical $-CH_2OH$ ou

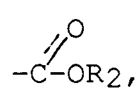

$R_2$ étant un atome d'hydrogène, un alkyle en $C_1$-$C_4$ avec éventuellement un ou plusieurs substituants pris parmi le fluor, le chlore, le brome et les groupes phényle, benzoyle, diméthylamino, diéthylamino, méthoxy et éthoxy, un cycloalkyle en $C_5$-$C_6$,un radical phényle, 1-naphtyle ou 2-naphtyle pouvant avoir chacun comme substituants de 1 à 3 atomes d'halogènes, un phényle, de 1 à 3 alkyles en $C_1$-$C_4$ ou un groupe chlorométhyle, fluorométhyle, trifluorométhyle, carboxyle, hydroxyle ou alcoxy en $C_1$-$C_4$, ou encore le groupe 2-furyle, 2-thiényle, 2-pyridyle, 3-pyridyle, 4-pyridyle, oxazolyle, thiazolyle, pyrimidinyle, pyridazinyle, 3-furyle, 3-thiényle ou 2-tétrazolyle, ou bien

$R_1$ est un radical

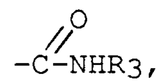

$R_3$ étant le radical d'un acide carboxylique ou sulfonique organique ayant de 1 à 15 atomes de carbone ou un radical $R_2$,

A représente un groupe $-CH_2-CH_2-$, $-CH=CH-$ trans ou $-C\equiv C-$,

W un groupe hydroxyméthylène libre ou bien éthérifié ou estérifié par un groupe tétrahydropyrannyle, tétrahydrofurannyle, $\alpha$-éthoxyéthyle, triméthylsilyle, diméthyl-tert-butylsilyle, tribenzylsilyle, acétyle, propionyle, butyryle ou benzoyle, ou un groupe

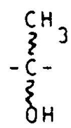

libre ou bien éthérifié ou estérifié par un groupe tétrahydropyrannyle, tétradrofurannyle, $\alpha$-éthoxyéthyle, triméthylsilyle, diméthyl-tert-butylsilyle, tribenzylsilyle, acétyle, propionyle, butyryle ou benzoyle, le groupe OH pouvant être en position $\alpha$ ou $\beta$,

D et E représentent ensemble une liaison directe ou bien

D est un alkylène linéaire, ramifié ou cyclique ayant de 1 à 10 atomes de carbone, avec éventuellement des atomes de fluor comme substituants, et

E un atome d'oxygène ou de soufre, une liaison directe, une liaison $-C=C-$ ou un groupe $-CR_6=CR_7-$, $R_6$ et $R_7$ étant différents l'un de l'autre et chacun un atome d'hydrogène ou de chlore ou un alkyle en $C_1$-$C_4$,

$R_4$ désigne un hydroxyle libre ou bien éthérifié ou estérifié par un groupe tétrahydropyrannyle, tétradrofurannyle, $\alpha$-éthoxyéthyle, triméthylsilyle, diméthyl-tert-butylsilyle, tribenzylsilyle, acétyle, propionyle, butyryle ou benzoyle, et

$R_5$ un atome d'hydrogène, un alkyle ou un alcényle pouvant avoir jusqu'à 6 atomes de carbone, éventuellement halogénés, un cycloalkyle en $C_3$-$C_6$, un groupe phényle, 1-naphtyle ou 2-naphtyle pouvant avoir chacun comme substituants de 1 à 3 atomes d'halogènes, un phényle, de 1 à 3 alkyles en $C_1$-$C_4$ ou un groupe chlorométhyle, fluorométhyle, trifluorométhyle, carboxyle, hydroxyle ou alcoxy en $C_1$-$C_4$, ou encore $R_5$ peut être le groupe 2-furyle, 2-thiényle, 2-pyridyle, 3-pyridyle, 4-pyridyle, oxazolyle, thiazolyle, pyrimidinyle, pyridazinyle, pyrazinyle, 3-furyle ou 3-thiényle,

ainsi que, si $R_2$ est un atome d'hydrogène, les sels de ces prostaglandines formés avec des bases compatibles sur le plan physiologique.

**2.** Procédé de préparation des 9-halogéno-prostaglandines de formul I selon la revendication 1, procédé caractérisé en ce que l'on fait réagir de manière connue un composé de formule II :

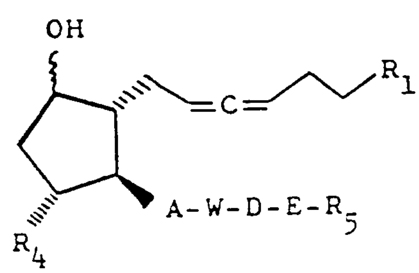

(II)

(dans laquelle le groupe 9-OH peut être à la position $\alpha$ ou $\beta$ et

$R_1$ est un radical

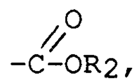

$R_2$ étant un alkyle en $C_1$-$C_4$ avec éventuellement un ou plusieurs substituants pris parmi le fluor, le chlore et le brome et les groupes phényle, benzoyle, diméthylamino, diéthylamino, méthoxy et éthoxy, un cycloalkyle en $C_5$-$C_6$ ou un groupe phényle, 1-naphtyle ou 2-naphtyle pouvant avoir chacun comme substituants de 1 à 3 atomes d'halogènes, un phényle, de 1 à 3 alkyles en $C_1$-$C_4$ ou un groupe chlorométhyle, fluorométhyle, trifluorométhyle, carboxyle, hydroxy ou alcoxy en $C_1$-$C_4$,ou encore un groupe 2-furyle, 2-thiényle, 2-pyridyle, 3-pyridyle, 4-pyridyle, oxazolyle, thiazolyle, pyrimidinyle, pyridazinyle, 3-furyle, 3-thiényle ou 2-tétrazolyle, ou bien $R_1$ est

un radical

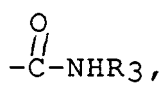

$R_3$ désignant un radical d'acide carboxylique ou sulfonique organique ayant de 1 à 4 atomes de carbone ou un radical $R_2$,

A, D et $R_5$ ayant les significations précédemment indiquées) après protection préalable des groupes OH des radicaux $R_2$, $R_4$ et W, avec la triphénylphosphine et du tétrachlorure de carbone ou de l'hexachloréthane ou avec le trifluorure de diéthylamino-soufre, ou bien après transformation du groupe hydroxy en 9 en groupe d'ester sulfonique, avec le fluorure de tétran-n-butylammonium, puis dans un ordre quelconque on libère les hydroxyles protégés et/ou on estérifie ou éthérifie des hydroxyles libres et/ou on hydrogène des doubles liaisons et/ou on saponifie un groupe carboxylique estérifié

($R_1$ = 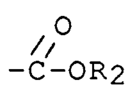)

et/ou on transforme un groupe carboxylique libre

($R_2$ = H) en un amide

(($R_1$ = 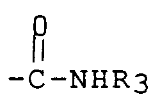)

et/ou on réduit un groupe

carboxylique libre ou estérifié

($R_1$ = 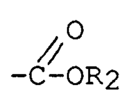).

**3.** Médicaments comprenant un ou plusieurs composés de la revendication 1 avec des adjuvants et véhicules courants.